# EUROPEAN PATENT APPLICATION

(11) **EP 4 233 561 A1**
(43) Date of publication of application: **30.08.2023**
(21) Application number: 21883096.6
(22) Date of filing: 12.10.2021
(51) Int. Cl.: A23L 29/00, A23L 19/00, A23L 27/30

(54) **METHOD FOR MANUFACTURING PLANT FERMENTED PRODUCT USING PLANT FERMENTED BROTH**

(30) Priority: 20.10.2020 KR 20200136301
(71) Applicant: Kang, Seung Chan, Jeju-si Jeju-do 63230 (KR)
(72) Inventor: Kang, Seung Chan, Jeju-si Jeju-do 63230 (KR)
(74) Representative: Isarpatent
(86) International application number: PCT/KR2021/014046
(87) International publication number: WO 2022/086025

(57) **Abstract**

The present invention provides a method for manufacturing a plant fermented product using a plant fermented broth, the method comprising: mixing 5 to 30 parts by weight of a first plant material, 100 parts by weight of water, 1 to 10 parts by weight of sugar, and 0.05 to 1 part by weight of lactic acid bacteria, and fermenting the resulting mixture at room temperature in a range of 5 to 25°C for 30 days to 6 months so that the material becomes acidic at pH 4 or less to obtain a first fermented broth; mixing 5 to 30 parts by weight of a second plant material, which is the same as or different from the first plant material, 100 parts by weight of water, 1 to 10 parts by weight of sugar, and 1 to 10 parts by weight of the first fermented broth, and fermenting the resulting mixture at room temperature in a range of 5 to 25°C for 30 days to 6 months so that the material has a sugar content in a range of 0.5 to 2 brix and becomes strongly acidic in a pH range of 2 to 4 to obtain a second fermented broth; and mixing a third plant material, which is the same as or different from the first plant material or the second plant material, with the second fermented broth at a weight ratio of 1:0.5 to 1:1.5, and fermenting the resulting mixture at room temperature in a range of 5 to 25°C for 20 days to 3 months to obtain a plant fermented product.

## Description

### TECHNICAL FIELD

The present invention relates to a novel fermentation method for various plant materials, which is a technology for obtaining a plant fermented broth by using lactic acid bacteria and sufficiently fermenting plant materials with the plant fermented broth at a low temperature to change plant polyphenols into a low molecular-weight state that can be absorbed.

### BACKGROUND ART

Humans burn oxygen and nutrients ingested from food to produce an energy source to sustain life. In this process, reactive oxygen species, which is extra oxygen, is also generated, which causes fatal harm to health by attacking and damaging normal cells and causing cell aging.

In cellular respiration, oxygen is essential for generating energy by completely oxidizing glucose to carbon dioxide and water. However, reactive oxygen species (free radicals) such as hydroxyl radicals (HO-) and peroxide anions (O²⁻) are incidentally generated in the course of operating the electron transport system of cellular respiration. Normal oxygen usually stays in the body for about 100 seconds or more, but reactive oxygen species, which is unstable, exists for one millionth to one billionth of a second and disappears instantaneously. As such, reactive oxygen species is a substance that exists in the blink of an eye, but it is so reactive to attack cell membranes, resulting in loss of cellular functions and damage to DNA. Since reactive oxygen species destroys the signal transduction system in cells or lowers immunity, it causes diabetes, arteriosclerosis, cancers, etc., and accelerates aging by preventing cell regeneration. In particular, age spots appearing on the skin of the elderly are caused by the action of reactive oxygen species. Because reactive oxygen species travels around the body well, it causes diseases in all organs, from blood vessels to joints. When reactive oxygen species stays in the body for a long time, it can cause chronic inflammation and thus become a big problem. Chronic inflammation, traveling all around the body, is known to be the source of all kinds of diseases. Chronic inflammation can increase the incidence of not only gastritis and arthritis but also vascular diseases such as stroke, arteriosclerosis, and myocardial infarction, as well as depression, dementia, and even cancers, and may cause sudden death.

On the other hand, all plants have their own physiologically active substances produced in themselves to protect themselves from the surrounding environment, which are polyphenols that exist in the form of various pigments. Polyphenols are a type of phytochemicals, which are plant chemicals. Phytochemicals are substances that plants make to protect themselves from the external environment (bacteria, pests, microorganisms, etc.) as they grow, and about 10,000 types have been identified so far. It is known that when introduced to human body, phytochemicals enhance human health through their powerful antioxidant actions. Among the numerous phytochemicals, the most powerful antioxidants are polyphenols. Polyphenols, which collectively refer to phenolic compounds in plants, are effective in cell protection, inflammation suppression, anti-aging, immunity improvement, and anticancer actions as they remove reactive oxygen species and chronic inflammation in the body with their antioxidant abilities. Polyphenols are effective on systemic health because of their antioxidant actions, and antioxidation means removing reactive oxygen species.

Polyphenols are formed during photosynthesis by plants, and the actual amount is extremely small compared to carbohydrates and proteins. Polyphenols are maximally formed when photosynthesis is active. At that time, the temperature exceeds 25°C, and at this temperature, polyphenols cannot be decomposed by bad bacteria or their own enzymes and so they can protect themselves. However, unlike carbohydrates and proteins, which are decomposed by digestive enzymes secreted from the salivary glands or gastric acid secreted from the stomach, polyphenols are difficult to digest and absorb. Polyphenol decomposition is closely related to temperature. The human body temperature is 36.5°C, which is too high for polyphenols to be decomposed, and there are no bacteria to decompose them at the temperature. Therefore, no matter how good polyphenols are, unless they are absorbed, they are excreted in feces. Consequently, polyphenols that are not decomposed by intestinal bacteria may not be ingestible.

As a commonly known technology for using plant fermentation, fruit syrup is made by mixing fruit and sugar at a ratio of 1:1, and after aging for a certain period of time, yeast is introduced for alcohol fermentation, water is added, acetic acid bacteria are mixed, and then the resulting mixture is fermented for about one week at a high temperature to complete fermented vinegar.

In addition, according to Patent Publication No. 10-2020-0062450, an antioxidant functional food composition containing fermented dandelion and fermented *Angelica keiskei,* or a mixture thereof as an active ingredient is proposed, wherein the fermented product is manufactured by a) inoculating *Lactobacillus plantarum* to dandelion, dandelion meal, *Angelica keiskei* or *Angelica keiskei* meal and then culturing at 30 ~ 37°C for 48 hours; b) drying the culture to a moisture content of 4 to 50; c) adding distilled water to the dried product and then extracting at 60 to 65°C for 2 to 3 hours; d) filtering the extract and then concentrating the same; and e) freeze-drying the concentrate.

In addition, according to Registered Patent No. 10-2065584, a method for producing a fermented tomato broth with enhanced total polyphenol, flavonoid and lycopene content and antioxidant and anti-obesity activity is proposed. The method comprises: (1) inoculating 0.8 to 1.2% (v/v) of *Lactobacillus plantarum* SRCM102223 strain (Accession Number: KFCC11811P) at a concentration of 106 to 108 CFU/ml to tomato juice; and (2) fermenting the tomato juice inoculated with the strain of (1) at 32 to 38°C for 44 to 52 hours.

As such, conventional technologies related to plant fermentation have limitations in decomposing polyphenols contained in plants to have low molecular weight, because an excessive amount of sugar is contained, the fermentation temperature is high, and the fermentation time is relatively short. As a result, it was difficult to improve the absorption of the fermented products of the plants to the body. In addition, there was a limitation in improving health by using various polyphenols from various plants.

### DETAILED DESCRIPTION OF THE INVENTION

### TECHNICAL PROBLEM

The present invention was conceived under the technical background described above, and a purpose of the present invention is to provide a method for manufacturing a plant fermented product with increased body absorption so as to obtain the functions and effects of various polyphenols contained in various plants.

Another purpose of the present invention is to provide a plant fermented product that is rich in physiologically active substances such as low molecular weight polyphenols and metabolites of lactic acid bacteria.

Another purpose of the present invention is to provide a method for manufacturing a novel plant fermented product that is capable of changing various ingredients of various plant groups into a state that can be absorbed by the human body and that can be mass-produced through a stable process.

Other purposes and technical features of the present invention will be presented in more detail in the following detailed description.

### TECHNICAL SOLUTION

To achieve the purpose described above, the present invention provides a method for manufacturing a plant fermented product using a plant fermented broth, the method comprising: mixing 5 to 30 parts by weight of a first plant material, 100 parts by weight of water, 1 to 10 parts by weight of sugar, and 0.05 to 1 part by weight of lactic acid bacteria, and fermenting the resulting mixture at room temperature in a range of 5 to 25°C for 30 days to 6 months so that the material becomes acidic at pH 4 or less to obtain a first fermented broth; mixing 5 to 30 parts by weight of a second plant material, which is the same as or different from the first plant material, 100 parts by weight of water, 1 to 10 parts by weight of sugar, and 1 to 10 parts by weight of the first fermented broth, and fermenting the resulting mixture at room temperature in a range of 5 to 25°C for 30 days to 6 months so that the material has a sugar content in a range of 0.5 to 2 brix and becomes strongly acidic in a pH range of 2 to 4 to obtain a second fermented broth; and mixing a third plant material, which is the same as or different from the first plant material or the second plant material, with the second fermented broth at a weight ratio of 1:0.5 to 1:1.5, and fermenting the resulting mixture at room temperature in a range of 5 to 25°C for 20 days to 3 months to obtain a plant fermented product.

In the present invention, after obtaining the second fermented broth, a fourth plant material, which is the same as or different from the first plant material or from the second plant material, may be mixed with the second fermented broth to obtain a third fermented broth, and then the third fermented broth may be mixed with the third plant material and fermented to obtain the plant fermented product.

In the present invention, as a plant material used in a process of obtaining the first fermented broth and the second fermented broth, any one selected from fresh plant material, dried plant material, hot water extract, and sugar-containing pickle may be mixed with each fermented broth.

In addition, in the present invention, the first fermented broth or the second fermented broth, in an initial step of long-term fermentation, after being mixed with a plant material, may be subjected to primary rapid fermentation at a high temperature by using a fermentation tank, and then to secondary long-term fermentation at room temperature.

In addition, the present invention provides a plant fermented product using a plant fermented broth prepared according to the method described above.

### ADVANTAGEOUS EFFECTS

According to the present invention, various plant materials that can be easily obtained from nature can be used in various states such as unprocessed raw materials, dried materials, powder, and sugar-pickled materials, and main parts of plants such as fruits, stems, leaves, roots, and peels can be easily utilized to obtain plant fermented products that are rich in absorbable polyphenols and various physiologically active substances produced by lactic acid bacteria.

The present invention allows mass production of high-quality plant fermented products from terrestrial and seabed plants through a stable process and is effective in distributing high-quality plant fermented products at low cost because advanced facilities are not required for fermentation process.

The plant fermented product using a plant fermented broth manufactured according to the present invention can be stored for a long time at room temperature, can be drunk as it is or mixed with cold or hot water to be diluted and ingested as tea or beverage, and has excellent absorption rate in the body so that plants that are difficult to ingest in a natural state may also be digested.

In particular, the plant fermented product according to the present invention allows the use of various functionalities that various plants have, including strong antioxidant action, antiseptic action, antibacterial action, anti-inflammatory action, catalytic action, and digestive action, and so can be applied to various fields such as pharmaceutical raw materials, cosmetic raw materials, food additives, plant activators, and disinfectants.

In addition, the plant fermented product using a plant fermented broth according to the present invention is not only excellent in digestion and absorption by the human bodybut also helps plant growth so that it can be used as a substitute for various fertilizers or pesticides and may help to increase the shelf-life of harvested plants . In particular, since it has an excellent capability to remove odorous components and thus provides a powerful deodorizing effect, it can help to remove odors and improve the environment at sites of livestock industry.

### DESCRIPTION OF THE DRAWINGS

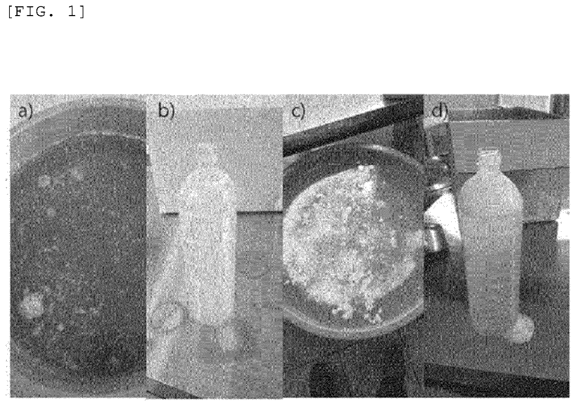

FIG. 1 shows the first fermented broth of the present invention, wherein a) is a photograph of *aDendropanax morbifera* Lev. fermented broth prepared in Example 1, b) is a photograph of a bottled *Dendropanax morbifera* Lev. fermented broth prepared in Example 1, c) is a photograph of a ginseng powder fermented broth prepared in Example 2, and d) is a photograph of a bottled ginseng fermented broth prepared in Example 2.

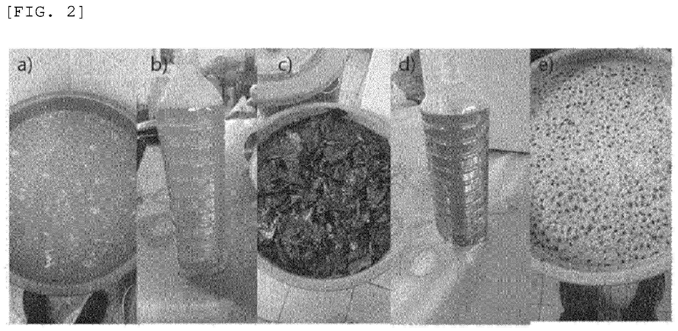

FIG. 2 shows the first fermented broth of the present invention, wherein a) is a photograph of a green tea fermented broth prepared in Example 3, b) is a photograph of a bottled green tea fermented broth prepared in Example 3, c) is a photograph of an *Ecklonia cava* fermented broth prepared in Example 6, d) is a photograph of a bottled black rice fermented broth prepared in Example 7, and e) is a photograph of a black sesame fermented broth prepared in Example 8.

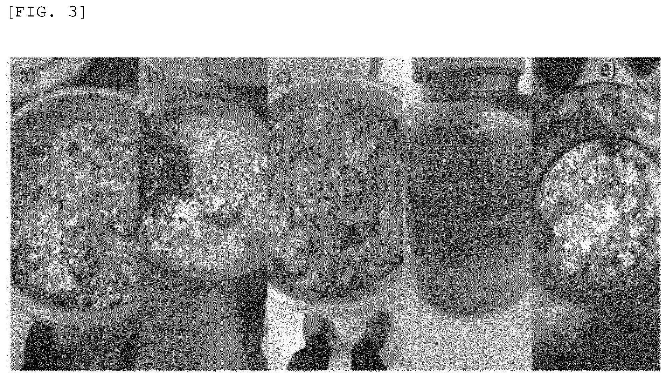

FIG. 3 shows the second fermented broth of the present invention, wherein a) is a photograph of a *Dendropanax morbifera* Lev. leaf fermented broth prepared in Example 9, b) is a photograph of a *Sargassum horneri* fermented broth prepared in Example 10, c) is a photograph of a *Ulva lactuca* fermented broth prepared in Example 11, d) is a photograph of a (turmeric + purple beet) fermented broth prepared in Example 12, and e) is a photograph of a balloon flower root fermented broth prepared in Example 14.

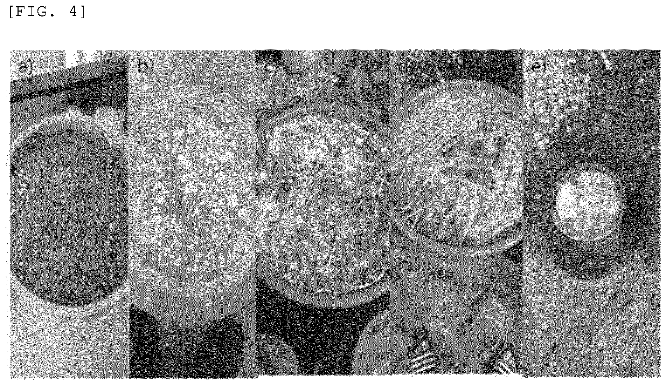

FIG. 4 shows the second fermented broth of the present invention, wherein a) is a photograph of a black sesame fermented broth prepared in Example 15, b) is a photograph of a ginseng fermented broth prepared in Example 16, c) is a photograph of a thistle fermented broth prepared in Example 17, d) is a photograph of a pine shoot fermented broth prepared in Example 18, and e) is a photograph of an onion fermented broth prepared in Example 19.

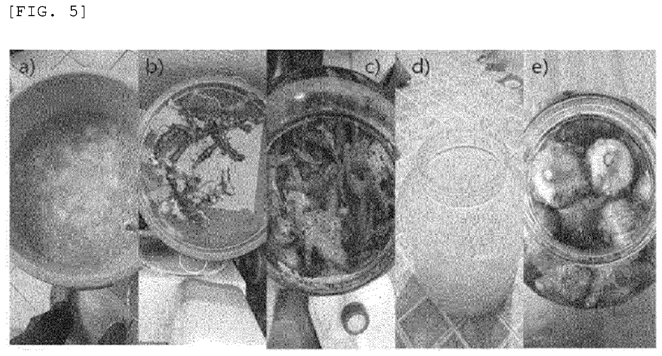

FIG. 5 shows the second fermented broth of the present invention, wherein a) is a photograph of an *Abelmoschus manihot* powder fermented broth prepared in Example 20, b) is a photograph of a *Smilax china* fermented broth prepared in Example 21, c) is a photograph of a honeyed wild ginseng fermented broth prepared in Example 22, d) ) is a photograph of a wild ginseng powder fermented broth prepared in Example 23, and e) is a photograph of a black garlic fermented broth prepared in Example 24.

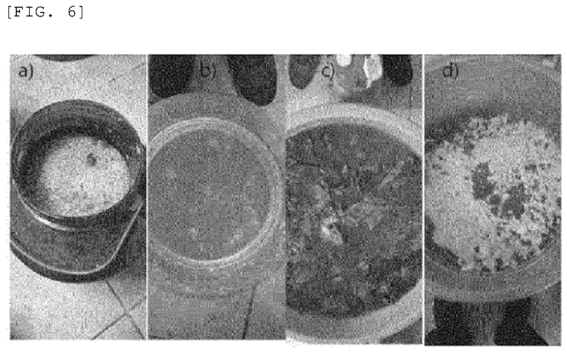

FIG. 6 shows the third fermented broth of the present invention, wherein a) is a photograph of fermenting *Usnea longissimi* in a *Dendropanax morbifera* Lev. fermented broth prepared in Example 25, b) is a photograph of fermenting balloon flower root powder in a *Dendropanax morbifera* Lev. fermented broth prepared in Example 26, c) is a photograph of *aUlva lactuca* fermented broth prepared in Example 27, and d) a photograph of a ginseng fermented broth prepared in Example 28.

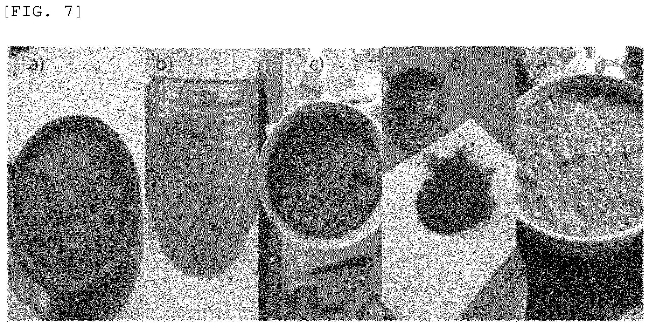

FIG. 7 shows the third fermented broth of the present invention, wherein a) is a photograph of a green tea powder fermented product prepared in Example 29, b) is a photograph of a green tea leaf fermented product prepared in Example 30, c) is a photograph of a steamed black rice fermented product prepared in Example 31, d) is a photograph of dried powder of a steamed black rice fermented product prepared in Example 32, and e) is a photograph of dried powder of a garlic husk fermented product prepared in Example 32.

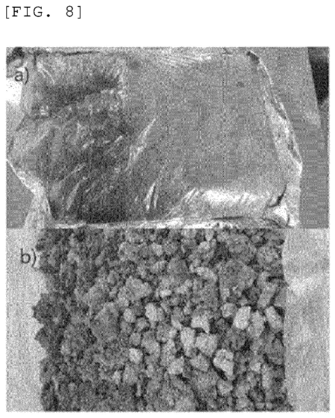

FIG. 8 shows the plant fermented product prepared in Example 35 of the present invention, wherein a) is a photograph of a barley sprout powder fermented product and b) is a photograph of dried produced of a barley sprout powder fermented product.

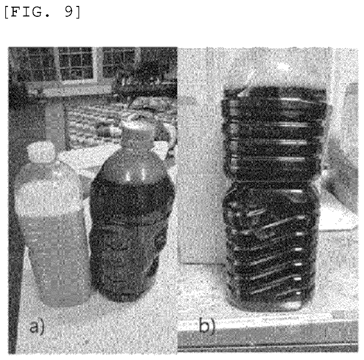

FIG. 9 shows the mixed fermented broth according to Experimental Examples 1 to 3, wherein a) is a photograph of is a mixed tangerine juice and a mixed red beet juice and b) is a photograph of a mixed black garlic extract.

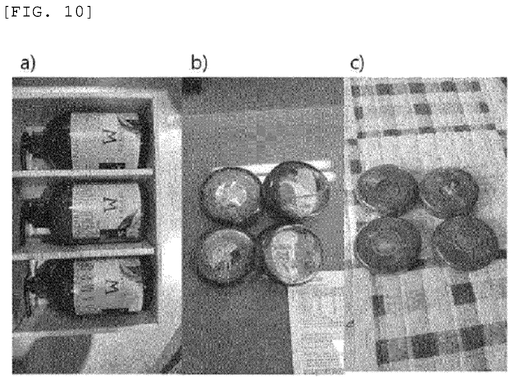

FIG. 10 shows a product from a fermented broth according to Experimental Examples 4 and 5 of the present invention, wherein a) is a photograph of shampoo and b) and c) are photographs of soap.

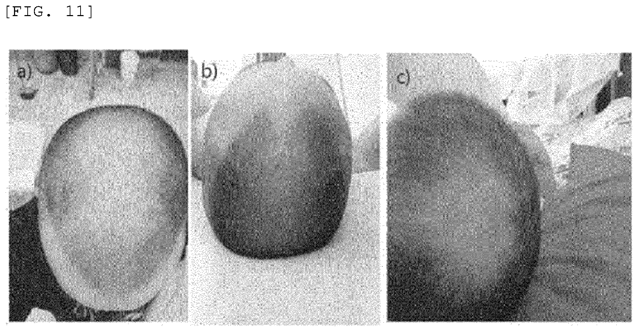

FIG. 11 shows photographs related to an experiment performed with a hair loss patient in relation to Experimental Examples 4 and 5 of the present invention, wherein a) is a photograph at the beginning of the experiment, b) is a photograph showing hair growth 2 months after the beginning of the experiment, and c) is a photograph showing more hair growth 3 months after the beginning of the experiment.

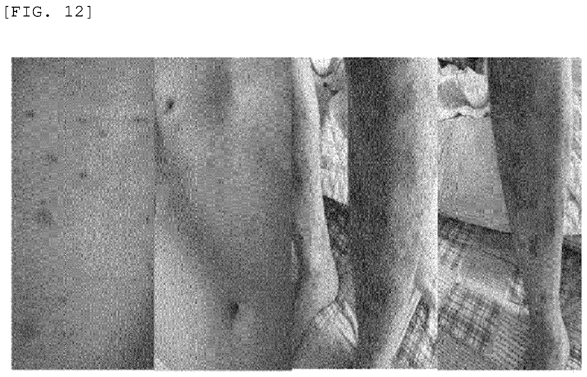

FIG. 12 is a photograph at the beginning of an experiment performed with a psoriasis patient according to Experimental Example 6 of the present invention.

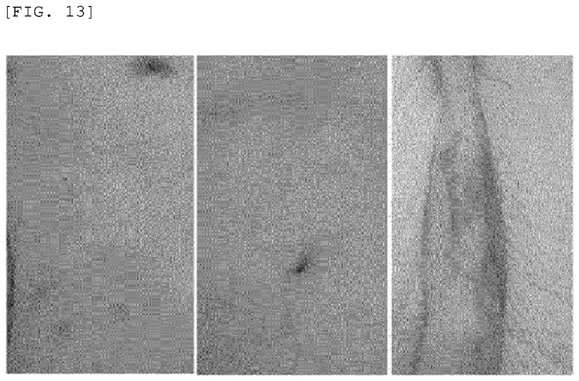

FIG. 13 is a photograph showing improvement 2 months after the beginning of the experiment with a psoriasis patient according to Experimental Example 6 of the present invention.

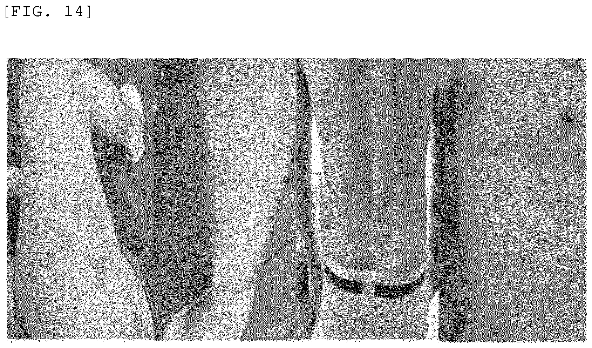

FIG. 14 is a photograph showing the clean body by the full recovery from psoriasis and regeneration of the necrotized skin 3 months after the beginning of the experiment with a psoriasis patient according to Experimental Example 6 of the present invention.

### BEST MODE

The method for manufacturing a plant fermented product using a plant fermented broth according to the present invention is characterized in that the method comprises: mixing 5 to 30 parts by weight of a first plant material, 100 parts by weight of water, 1 to 10 parts by weight of sugar, and 0.05 to 1 part by weight of lactic acid bacteria, and fermenting the resulting mixture at room temperature in a range of 5 to 25°C for 30 days to 6 months so that the material becomes acidic at pH 4 or less to obtain a first fermented broth; mixing 5 to 30 parts by weight of a second plant material, which is the same as or different from the first plant material, 100 parts by weight of water, 1 to 10 parts by weight of sugar, and 1 to 10 parts by weight of the first fermented broth, and fermenting the resulting mixture at room temperature in a range of 5 to 25°C for 30 days to 6 months so that the material has a sugar content in a range of 0.5 to 2 brix and becomes strongly acidic in a pH range of 2 to 4 to obtain a second fermented broth; and mixing a third plant material, which is the same as or different from the first plant material or the second plant material, with the second fermented broth at a weight ratio of 1:0.5 to 1:1.5, and fermenting the resulting mixture at room temperature in a range of 5 to 25°C for 20 days to 3 months to obtain a plant fermented product.

### MODE OF THE INVENTION

The present invention relates to a method for producing a plant fermented product using a plant fermented broth, and proposes a method for converting plant polyphenols into absorbable states with a low molecular weight through a multi-fermentation process by using a plant fermented broth obtained by fermentation with lactic acid bacteria, and a plant fermented product having low-molecular weight polyphenols manufactured thereby.

The inventors of the present invention repeatedly performed studies to effectively use useful physiologically active components of plants such as polyphenols, and completed the present invention by confirming that physiologically active components can be used more effectively when a fermented product containing low-molecular weight polyphenols and medicinal ingredients is manufactured by sufficiently fermenting plants with lactic acid bacteria at a low temperature for a long time.

Since polyphenols cannot be digested and absorbed by humans, no matter how much polyphenols are ingested, they are ineffective. However, it was confirmed that when plants are sufficiently fermented with lactic acid bacteria for a long time, polyphenol molecules are decomposed and the absorption in the body is improved. In addition, it was also confirmed that during the fermentation process, physiologically active substances such as enzymes, organic acids, and bacteriocins, which are metabolites of lactic acid bacteria, are produced and they are effective in killing pathogens such as viruses. In addition, it was confirmed that fermentation should be performed with lactic acid bacteria at a low temperature for a long time in order to decompose polyphenols and improve the absorption in the body.

Fermenting plants with lactic acid bacteria has an advantage of improving preservation due to the excellent storability. In addition, as polyphenols of plants are decomposed into low-molecular substances during the fermentation process, the absorption rate is increased and various physiologically active components are produced, and so fermentation is more useful for health. Plant fermentation using lactic acid bacteria requires a long time and a technology in the fermentation and aging process. Even when the same method and the same process are used, decay or alcohol fermentation may happen due to the temperature, humidity, light, airborne microorganisms. Mass production of a plant fermented product has not been accomplished yet to convert polyphenols to an absorbable low molecular weight level.

In the present invention, plant materials are fermented with lactic acid bacteria to maintain strong acidity in the range of pH 2 to pH 4, and a plant fermented broth that is in strong fermenting bacteria surviving this process, low-molecular-weight polyphenols, and various physiologically active substances produced by the fermenting bacteria is prepared. Then, the plant fermented broth is fermented with a plant material to manufacture a final plant fermented product.

The present invention consists of a process described below.

First, as a process of preparing a fermented broth, a subject material is prepared as a plant material for preparing a fermented broth from among terrestrial plants or seaweeds, and the fermented broth material is fermented with lactic acid bacteria to prepare a fermented broth. Specifically, 5 to 30 parts by weight of a first plant material, 100 parts by weight of water, 1 to 10 parts by weight of sugar, and 0.05 to 1 part by weight of lactic acid bacteria are mixed, and the resulting mixture is fermented at room temperature in a range of 5 to 25°C for 30 days to 6 months so that the material becomes acidic at pH 4 or less to obtain a first fermented broth.

A plant materials, water, sugar, which is food for lactic acid bacteria, and lactic acid bacteria are mixed and put into in a fermenter, and fermented sufficiently at a low temperature the pH drops below 4. A first fermented broth, containing strong fermenting bacteria surviving the strong acidic conditions, low-molecular-weight polyphenols, and various organic acids produced by the fermenting bacteria, can be a product as it is, but as it has strong antibacterial, antiviral, and antioxidant activities, it can be used as strong fermenting bacteria in subsequent processes.

The first plant material used in the present invention may be used alone or in combination of several types. Edible plants or non-edible plants can be used as plant materials. As edible plants, not only terrestrial plants such as fruits, vegetables, grains, and medicinal materials, but also seaweeds such as kelp, sea mustard, laver, hijiki, and green laver can be used. In addition, non-edible materials, including not only terrestrial plants such as cypress, ginkgo, bush clover, and paper mulberry as well as seaweeds such as *Ecklonia cava* and *Sargassum horneri,* can be used.

As a next step, 5 to 30 parts by weight of a second plant material, which is the same as or different from the first plant material, 100 parts by weight of water, 1 to 10 parts by weight of sugar, and 1 to 10 parts by weight of the first fermented broth are mixed, and the resulting mixture is fermented at room temperature in a range of 5 to 25°C for 30 days to 6 months so that the material has a sugar content in a range of 0.5 to 2 brix and becomes strongly acidic in a pH range of 2 to 4 to obtain a second fermented broth.

In the present invention, instead of directly fermenting a final plant material with lactic acid bacteria, a fermented broth may firstly be obtained and used to finally ferment a plant material or a multi-fermentation process may be carried out in which a first fermented broth obtained by using lactic acid bacteria is mixed with a plant material, and the resulting mixture is fermented to obtain a second fermented broth, which is then mixed with a final plant material and fermented. The multi-fermentation process may increase the degree of decomposition of polyphenols included in the final plant material to improve the digestion and absorption. In particular, by mixing and fermenting a second fermented broth with a third plant material, miscellaneous germs can be removed by the sterilizing power or disinfecting power of a highly acidic second fermented brothand the fermentation efficiency of a third plant material can beincreased. As a result, the fermentation odor of a final plant fermented product may be improved, and the color may be turned clear and transparent.

In addition, in a multi-fermentation process, the type and state of each plant material may be changed to, for example, raw material, dried material, powder, and pre-treated material so that unique and changed components of the plant material can evenly included in the final plant fermented product. Therefore, the medicinal properties of the used plant material but also the physiologically active substances that are beneficial to human body can be retained to be absorbable.

As a next step, a third plant material, which is the same as or different from the first plant material or the second plant material, is mixed with the second fermented broth at a weight ratio of 1:0.5 to 1:1.5, and the resulting mixture is fermented at room temperature in a range of 5 to 25°C for 20 days to 3 months to obtain a plant fermented product.

With regard to mixing of a plant material and a second fermented broth, to obtain, for example, a paste-like fermented product, a second fermented broth may be mixed in a fermentation vessel such that a plant material is immersed. A plant material may be used alone or in combination with several types. In addition, according to the intended use, water and sugar may be additionally mixed to adjust the concentration of the final plant fermented product.

In the present invention, after obtaining a second fermented broth, a fourth plant material, which is the same or different from a first plant material or a second plant material, may be mixed with the second fermented broth to obtain a third fermented broth, and the third fermented broth may be mixed with the third plant material and fermented to obtain the plant fermented product. Similar to the fermented broth and the second fermented broth described above, the third fermented broth may undergo a fermentation process for fermenting at room temperature in a range of 5 to 25°C for 30 days to 6 months, or the third fermented broth may be completed simply by mixing. In the latter case, before obtaining a final plant fermented product by mixing with a third plant material, an aging step of storing at room temperature or refrigerating may be performed.

In the present invention, a plant material used in the process of obtaining the first fermented broth and the second fermented broth may be mixed as any one selected from fresh raw material, dried product, powder, hot water extract and sugar-containing pickle.

All plants contain polyphenols in the form of pigments. Representative polyphenols include beta-carotene, lycopene, lutein, zeaxanthin, flavonoid, ellagic acid, quercetin, lutin, curcumin, hesperetin, anthocyanin, isoflavone, lignan, beta-sitosterol, saponin, catechin, indol, sulforaphane, and capsaicin.

These various polyphenols are found in fruits such as mango, banana, tomato, watermelon, guava, persimmon, dried persimmon, yellow peach, grapefruit, kiwi, melon, pomegranate, plum, raspberry, grape, blueberry, orange, lemon, tangerine, citron, lime, calamansi, avocado, noni, pomegranate, and cranberry; vegetables such as carrot, sweet potato, kale, pumpkin, paprika, cabbage, spinach, asparagus, broccoli, corn, saffron, turnip, kale, mustard, collard, red pepper, eggplant, sesame seed, radish, Chinese cabbage, garlic, celery, and parsley; seaweeds such as kelp, green laver, sea mustard, and hijiki; grains such as barley, rice, black rice, buckwheat, millet, sorghum, oats, black bean, black sesame seed, and Chinese matrimony vine; and bulbs (root plants) such as ginseng, bellflower root, peanut, turmeric, ginger, *Coconopsis lanceolata,* and *Pueraria thunbergiana.*

Therefore, a plant material used in a plant fermented broth or a plant fermented product of the present invention may include various terrestrial and seabed plants, and may include various plant materials, including plant leaves, stems, fruits, roots, etc., in particular, *Dendropanax morbifera* Lev. leaves, green tea leaves, mulberry leaves or peels of various vegetables and fruits, nuts, seeds, ginkgo, rice bran, bamboo shoot, and corn cob.

The first fermented broth or the second fermented broth, in an initial step of long-term fermentation, after being mixed with a plant material, may be subjected to primary rapid fermentation at a high temperature by using a fermentation tank, and then to secondary long-term fermentation at room temperature.

The plant fermented product manufactured according to a multi-fermentation process of the present invention is capable of sufficiently fermenting a plant material at a low temperature with the strong power of the plant fermented broth containing strongly acidic water at pH3, fermentation bacteria that have survived harsh conditions, enzymes, various organic acids, and low-molecular weight polyphenols. In this process, a plant material is changed to a plant fermented product that is rich in low-molecular polyphenols and various physiologically active substances produced by the fermentation bacterial.

In addition, the fermented product manufactured by the present invention has further health benefit, as the nutrients and pharmacological components of the fermented broth are added to the nutrients and pharmacological components of plant material and the diversity and amount of the nutrients and pharmacological components are increased with various physiologically active substances produced by lactic acid bacteria. In addition, by varying the combination of a plant material and a fermented broth, various types of fermented products can be easily manufactured. For example, when green tea is fermented with a *Dendropanax morbifera* Lev. fermented broth, a green tea-*Dendropanax morbifera* Lev. fermented product is manufactured, and when green tea is fermented with a ginseng fermented broth, a green tea- ginseng fermented product is manufactured. Since the plant fermented product of the present invention has excellent pharmacological activity, it can be used in various fields. For example, a paste-like fermented product manufactured according to the present invention can be used by mixing it with water or by brewing the active ingredients in water, and in some cases, the product can be formulated as a pill. Therefore, a paste-like fermented product has an advantage of being usable in various forms compared to a liquid fermented product.

Hereinafter, preparation of a plant fermented broth according to the present invention and a manufacturing process of a plant fermented product using the same will be presented in detail through specific Examples, and the effect of a manufactured plant fermented product will be additionally described.

### Examples

### Preparation of a first fermented broth

To prepare a first fermented broth, a first plant material was selected and mixed with water, sugar, and lactic acid bacteria, and the resulting mixture was fermented. The first plant materialsused and their content are shown in Table 1, and 100 parts by weight of water, 5 parts by weight of sugar, and 1 part by weight of *Lactococcus lactis* subsp. lactis were inoculated as a fermentation bacteria and fermented at room temperature for 30 days. After the fermentation process was completed, only the liquid phase was obtained to prepare a fermented broth. FIGS. 1 and 2 show photographs of the first fermented broth according to each Example.

| | First plant material | Content |
|---|---|---|
| Example 1 | *Dendropanax morbifera* Lev. | 20 parts by weight |
| Example 2 | Ginseng powder | 5 parts by weight |
| Example 3 | Green tea leaf | 20 parts by weight |
| Example 4 | *Ulva lactuca* | 20 parts by weight |
| Example 5 | Kelp | 20 parts by weight |
| Example 6 | *Ecklonia cava* | 20 parts by weight |
| Example 7 | Black rice | 20 parts by weight |
| Example 8 | Black sesame | 20 parts by weight |

### Preparation of a second fermented broth

As a secondary fermented broth, any one of the first fermented broth according to Example described above was used in place of lactic acid bacteria, and mixed with a second plant material, water, and sugar to prepare a second fermented broth. The first fermented broth and the second fermented broth used are described in Table 2 below, and 20 parts by weight of the second plant material, 100 parts by weight of water, 5 parts by weight of sugar, and 5 parts by weight of the first fermented broth were inoculated and fermented at room temperature for 30 days. Then, the second fermented broth was completed by separating and obtaining only the liquid phase. FIGS. 3 to 5 show photographs of the second fermented broth according to each Example.

A final plant fermented product may be manufactured by mixing the plant material with the first fermented broth described above, but in the present invention, a multi-fermentation process is carried out in which a second plant fermented product is prepared and mixed with a plant material to be fermented so that the ingredients and functions of a final plant fermented product may be further improved.

| | Second plant material | First fermented broth |
|---|---|---|
| Example 9 | *Dendropanax morbifera* Lev. leaf | *Dendropanax morbifera* Lev. fermented broth |
| Example 10 | *Sargassum horneri* | *Dendropanax morbifera* Lev. fermented broth |
| Example 11 | *Ulva lactuca* | *Dendropanax morbifera* Lev. fermented broth |
| Example 12 | Turmeric + purple beet | *Dendropanax morbifera* Lev. fermented broth |
| Example 13 | *Ecklonia cava* | Ginseng fermented broth |
| Example 14 | Balloon flower root | Ginseng fermented broth |
| Example 15 | Black sesame | Ginseng fermented broth |
| Example 16 | Ginseng powder | Ginseng fermented broth |
| Example 17 | Thistle | *Ulva lactuca* fermented broth |
| Example 18 | Pine shoot | *Ulva lactuca* fermented |
| | | broth |
| Example 19 | Onion | *Ulva lactuca* fermented broth |
| Example 20 | *Abelmoschus manihot* powder | *Ulva lactuca* fermented broth |
| Example 21 | *Smilax china* | Black rice fermented broth |
| Example 22 | Honeyed ginseng | Black rice fermented broth |
| Example 23 | Wild ginseng powder | Black rice fermented broth |
| Example 24 | Black garlic | Black rice fermented broth |

### Preparation of a third fermented broth

A third fermented broth with added medicinal properties was prepared by mixing a fourth plant material that was different from a first plant material or a second plant material. The *Dendropanax morbifera* Lev. fermented broth prepared according to Example 9 was used as a second fermented broth and the various raw materials shown in Table 3 were mixed as a fourth plant material to prepare a third fermented broth. In contrast to the Examples described above, a low-temperature long-term fermentation process was not carried out, and the third fermented broth was prepared only by a mixing process at room temperature. The completed third fermented broth was stored at room temperature or refrigerated. FIGS. 6 and 7 show photographs of the third fermented broth according to each Example.

| | Fourth plant material | Second fermented broth |
|---|---|---|
| Example 25 | *Usnea longissimi* powder | *Dendropanax morbifera* Lev. fermented broth |
| Example 26 | Balloon flower root | *Dendropanax morbifera* Lev. fermented broth |
| Example 27 | *Ulva lactuca* | *Dendropanax morbifera* Lev. fermented broth |
| Example 28 | Ginseng powder | *Dendropanax morbifera* Lev. fermented broth |
| Example 29 | Green tea powder | *Dendropanax morbifera* Lev. fermented broth |
| Example 30 | Green tea leaf | *Dendropanax morbifera* Lev. fermented broth |
| Example 31 | Steamed black rice | *Dendropanax morbifera* Lev. fermented broth |
| Example 32 | Garlic husk | *Dendropanax morbifera* Lev. fermented broth |
| Example 33 | Kelp powder | *Dendropanax morbifera* Lev. fermented broth |

### Preparation of final plant fermented product

The fermented broth prepared according to the Examples described above was mixed with a third plant material, which was the same as or different from the first plant material or the second plant material, and the resulting mixture was aged at room temperature to prepare a fermented product in a paste state. Five liters of the fermented broth and 5 liters of a third plant material were put into a fermenter, and mixed by stirring well. Then, the resulting mixture was aged at room temperature in a range of 5°C to 25°C depending on the season for 20 to 30 days to complete a final fermented product. The types of the third plant material and the fermented broth used are shown in Table 4. FIG. 8 shows a photograph of the plant fermented product according to Example 35 as an example.

| | Third plant material | Fermented broth |
|---|---|---|
| Example 34 | *Usnea longissimi* powder | Ginseng fermented broth (Example 2) |
| Example 35 | Balloon flower root | *Dendropanax morbifera* Lev. fermented broth (Example 1) |
| Example 36 | *Ulva lactuca* | Green tea fermented broth (Example 3) |
| Example 37 | Ginseng powder | *Dendropanax morbifera* Lev. fermented broth (Example 9) |
| Example 38 | Green tea powder | *Dendropanax morbifera* Lev. fermented broth (Example 25) |

### Experimental Examples

Experimental Example 1 - 3: Improvement of juice preservability
a) Tangerine was juiced, and the *Dendropanax morbifera* Lev. fermented broth prepared in Example 9, sterilized by boiling, was mixed with the juice at a content of 5 wt% to observe changes. Decay did not happen for 15 days at room temperature and 30 days in a refrigerator, and the texture became soft as the tissue of the tangerines was decomposed. Carbon dioxide was generated from the juice mixture over time, but decay did not happen even after six months.
b) Red beet was juiced and subject to an experiment in the same way as the tangerine juice, and the results showed that long-term preservation was maintained without decay.
c) Black garlic was water-extracted and subject to an experiment in the same way as the tangerine juice, and the results showed that long-term preservation was maintained without decay.

FIG. 9 shows a photograph of the juice mixture according to Experimental Example 1 to 3.

### Experimental Examples 4 and 5: Hair loss improvement by shampoo and soap prepared by mixing a fermented broth

a) A mixed shampoo was prepared by adding 5% of the *Dendropanax morbifera* Lev. fermented broth according to Example 9 to shampoo raw materials. Long-term use of the mixed shampoo showed disappearance of a scalp disease and prevention of hair loss.
b) Eight types of fermented broths prepared according to Examples 9, 10, 11, 12, 13, 14, 15, and 16 were mixed at the same proportion, and 3% of the fermented broth mixture was added to prepare soap. The soap from the fermented broth mixture was applied to face and scalp washing for a long time, and the results showed that a scalp disease disappeared, and hair growth began to occur on the scalp of a hair loss patient after 3 months.

FIG. 10 shows a photograph offermented broth-mixed shampoo and fermented broth-mixed soap, and FIG. 11 shows a photograph representing the improvement of hair loss according to Experimental Examples 4 and 5.

### Experimental Example 6

Eight fermented liquids prepared according to Examples 9, 10, 11, 12, 13, 14, 15, and 16 were mixed at the same proportion, and a small amount of the fermented broth mixture was ingested by a psoriasis patient in the 50s and applied to the skin. The results confirmed that after one month, the skin was improved, and after 3 months, psoriasis was completed cured. FIGS. 12 to 14 show the improved appearance of the psoriasis patient according to Experimental Example 6.

Although the present invention has been illustratively described through preferable Examples above, the present invention is not limited to such specific Examples, and may be modified, changed, or improved to various forms provided within the scope of the technical principles presented in the present invention, specifically, the claims.

### INDUSTRIAL APPLICABILITY

As described above, the present invention provides a method for manufacturing a plant fermented product using a plant fermented broth so that the manufactured plant fermented product may be stored for a long time at room temperature and can be drunk as it is or mixed with cold or hot water to be ingested as diluted tea or beverage. In addition, due to the high absorption rate in the body, the product allows the digestion of plants that are difficult to be ingested in the natural state. The plant fermented product allows the utilization of various functionalities of various plants, including powerful antioxidant activity, antiseptic activity, antibacterial activity, anti-inflammatory activity, catalytic activity, and digestive activity, and so it can be applied to various fields, including pharmaceutical raw materials, cosmetic raw materials, food additives, plant activators, and disinfectants. The plant fermented product using a plant fermented broth is not only excellent in digestion and absorption by the human body but also helps plant growth so that it can be used as a substitute for various fertilizers or pesticides and may help to increase the shelf-life of harvested plants. In particular, since it has an excellent capability to remove odorous components and thus provides a powerful deodorizing effect, it can help to remove odors and improve the environment at sites of livestock industry. Therefore, the invention of the present application has very high industrial applicability.

## Claims

1. A method for manufacturing a plant fermented product using a plant fermented broth, the method comprising:
mixing 5 to 30 parts by weight of a first plant material, 100 parts by weight of water, 1 to 10 parts by weight of sugar, and 0.05 to 1 part by weight of lactic acid bacteria, and fermenting the resulting mixture at room temperature in a range of 5 to 25°C for 30 days to 6 months so that the material becomes acidic at pH 4 or less to obtain a first fermented broth;
mixing 5 to 30 parts by weight of a second plant material, which is the same as or different from the first plant material, 100 parts by weight of water, 1 to 10 parts by weight of sugar, and 1 to 10 parts by weight of the first fermented broth, and fermenting the resulting mixture at room temperature in a range of 5 to 25°C for 30 days to 6 months so that the material has a sugar content in a range of 0.5 to 2 brix and becomes strongly acidic in a pH range of 2 to 4 to obtain a second fermented broth; and
mixing a third plant material, which is the same as or different from the first plant material or the second plant material, with the second fermented broth at a weight ratio of 1:0.5 to 1:1.5, and fermenting the resulting mixture at room temperature in a range of 5 to 25°C for 20 days to 3 months to obtain a plant fermented product.

2. The method for manufacturing a plant fermented product using a plant fermented broth according to claim 1, wherein after obtaining the second fermented broth, a fourth plant material, which is the same or different from a first plant material or a second plant material, is mixed with the second fermented broth to obtain a third fermented broth, and the third fermented broth is mixed with the third plant material and fermented to obtain the plant fermented product.

3. The method for manufacturing a plant fermented product using a plant fermented broth according to claim 1, wherein as a plant material used in a process of obtaining the first fermented broth and the second fermented broth, any one selected from fresh plant material, dried plant material, hot water extract, and sugar-containing pickle is mixed.

4. The method for manufacturing a plant fermented product using a plant fermented broth according to claim 1, wherein the first fermented broth or the second fermented broth, in an initial step of long-term fermentation, after being mixed with a plant material, is subjected to primary rapid fermentation at a high temperature by using a fermentation tank, and then to secondary long-term fermentation at room temperature.

5. A plant fermented product using a plant fermented broth manufactured by a method according to any one of claims 1-4.
